# EUROPÄISCHE PATENTANMELDUNG

(11) **EP 0 584 585 A1**
(43) Veröffentlichungstag der Anmeldung: **02.03.1994**
(21) Anmeldenummer: 93112321.0
(22) Anmeldetag: 31.07.1993
(51) Int. Cl.: C07C 231/14, C07C 233/47

(54) **Verfahren zur Herstellung von N-Acylderivaten der 5-Aminolävulinsäure sowie des Hydrochlorids der freien Säure**

(30) Priorität: 24.08.1992 DE 4228084
(71) Anmelder: SÜDZUCKER AKTIENGESELLSCHAFT MANNHEIM/OCHSENFURT, D-68165 Mannheim (DE)
(72) Erfinder: Descotes, Gérard, F-69006 LYON (FR); Cottier, Louis, F-69300 Caluire (FR); Eymard, Laurent, F-26120 Malissard (FR); Rapp, Knut M., D-67591 OFFSTEIN (DE)
(74) Vertreter: Gleiss, Alf-Olav, Dr.jur. Dipl.-Ing.

(57) **Zusammenfassung**

Die Erfindung betrifft ein Verfahren zur Herstellung von N-Acylderivaten der 5-Aminolävulinsäure der allgemeinen Formel R-CO-NH-CH₂-CO-CH₂-CH₂-COOH, in der R Methyl, Ethyl, Propyl, Isopropyl, Butyl, Phenyl, Benzyl, Furyl oder Furfuryl bedeuten, sowie des Hydrochlorids der freien Säure durch saure Hydrolyse dadurch gekennzeichnet, daß man 5-Hydroxymethylfurfural mit einem Nitril in saurer Lösung kondensiert und die erhaltene N-Acylaminomethylfurfuralverbindung durch Photooxidation in ein N-Acyl-5-aminomethyl-5-hydroxydihydro-2,5-furan-2-on überführt und dieses mit Zink in Essigsäure unter Ultraschall zu der N-Acyl-5-aminolävulinsäure reduziert, wobei ferner durch saure Hydrolyse das Hydrochlorid der 5-Aminolävulinsäure erhalten wird.

## Beschreibung

Die Erfindung betrifft ein Verfahren zur Herstellung von N-Acylderivaten der 5-Aminolävulinsäure sowie des Hydrochlorids der freien, also unsubstituierten Säure durch saure Hydrolyse.

Die δ-Aminolävulinsäure oder 5-Aminolävulinsäure, bzw. 5-Amino-4-oxo-pentansäure (ALA) ist ein Vorläufer von Vitamin B₁₂, Häm und Chlorophyll und ist einmal zur Biosynthese von Vitamin B₁₂ geeignet und zum anderen auch mit ihren Derivaten als photodynamisches Herbizid von Interesse.

Verfahren zur Herstellung von ALA als Hydrochlorid sind ausgehend von Tetrahydrofurfurylamin oder Furfural von H. Kawakami et al. in Agric. Biol. Chem., 1991, 56, 1687-8 und von K. Suzuki et al. in JA-03072450-A 2 beschrieben. Die Ausbeuten sind bei diesen mehrstufigen Verfahren verhältnismäßig gering.

Die Herstellung von ALA als Hydrochlorid durch saure Hydrolyse anderer Heterocyclen wie Piperidin-2,5-dion ist von C. Herdeis et al. in Arch. Pharm., 1984, 317, 304-306 beschrieben. Durch Hydrolyse von Oxazolinon erhält man ALA nach W. Shen et al. in Youji Huaxue, 1987, 278-80 bzw. nach N.I. Aronova et al. bzw. das Hydrochlorid von ALA gemäß DE-22 08 800-A2 und Izv. Akad. Nauk SSSR, Ser. Khim, 1973, 657-8. Die gleiche saure Hydrolyse von Ketoestern, welche durch Kondensation von Hippursäure mit einem Anhydrid hergestellt wurden, ist von D. Evans et al. in J. Chem. Soc., Chem. Comm., 1978, 753-4 beschrieben, wobei die Reaktion jedoch die Bildung eines Dianions unter wasserfreien Bedingungen erfordert. Das Hydrochlorid von ALA wird in gleicher Weise durch saure Hydrolyse von N-Phthalimidoderivaten der ALA erhalten, welche im allgemeinen durch Kondensation eines Salzes des Phthalimids mit einem δ-Brom-Derivat erhalten werden; (S. I. Zav-'yalov et al., Izv. Akad. Nauk SSSR, Ser. Khim., 1987, 1796-9; E. Benedikt et al., Z. Naturforsch., B: Anorg. Chem., Org. Chem., 1986, 41B, 1593-4; Z. Rykowski et al., Pol., 1979, PL 104118 und Rocz. Chem., 1977, 51, 1675-8).

Die Reduktion von Nitrosylderivaten nach Z. Chabudzinski et al. in Chem. Stosow., 1977, 21, 251-5 oder von Acylcyaniden nach A. Pfaltz et al. in Tetrahedron Lett., 1984, 25, 2977-80 führt ebenfalls zum Hydrochlorid des ALA.

Biosynthetische Verfahren zur Herstellung von ALA unter verschiedenen Kulturbedingungen sind beschrieben von S. Nagai et al. in JP 02261389-A2; Koesnandar in Biotechnol. Lett., 1989, 11, 567-72 und von T. Sasaki et al. in JP 0292293-A2; diese Methoden erfordern jedoch sehr verdünnte Lösungen und ergeben nur geringe Konzentrationen von ALA.

Aufgabe der Erfindung ist es, ein einfaches Verfahren zur Herstellung von ALA und deren Derivaten in guter Ausbeute und Reinheit zur Verfügung zu stellen, wobei das Ausgangsprodukt von allgemein wohlfeil zur Verfügung stehenden Zuckern erhalten wird.

Zur Lösung dieser Aufgabe dient ein Verfahren zur Herstellung von N-Acylderivaten der ALA der allgemeinen Formel I

R - CO - NH - CH₂ - CO - CH₂ - CH₂ - COOH (I),

in welcher R eine niedere Alkylgruppe mit 1 bis 10 C-Atomen, insbesondere eine Methyl-, Ethyl-, Propyl-, Isopropyl- oder Butylgruppe oder eine Phenyl-, Benzyl-, Furyl- oder Furfuryl-Gruppe bedeuten, wie es im Kennzeichen des Hauptanspruches dargestellt ist, bzw. des Hydrochlorids der freien Säure durch saure Hydrolyse gemäß Unteranspruch 2, wobei bevorzugte Verfahrensmaßnahmen in den weiteren Unteransprüchen erläutert sind.

Das Verfahren läuft in drei Schritten ab und geht aus von 5-Hydroxymethylfurfural (HMF), welches durch sauren Abbau von Zuckern erhalten wird.

In der ersten Stufe wird HMF der Formel II 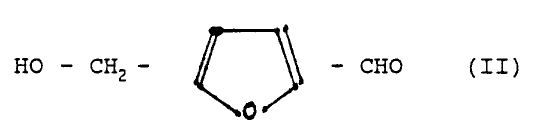
durch Kondensation mit einem Nitril der Formel III

R - C ≡ N (III),

in welcher der Rest R die oben angegebene Bedeutung hat, in das entsprechende N-Acyl-5-aminomethylfurfural der Formel IV überführt: 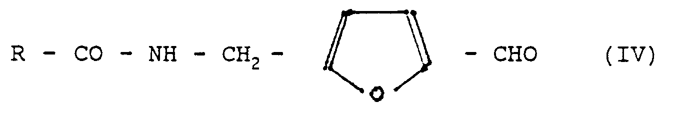
Die durch Säure katalysierte Kondensation erfolgt vorzugsweise bei einer Temperatur von 0 bis 50° C. Als Säure wird eine starke Säure, vorzugsweise eine Mineralsäure wie Schwefelsäure oder Phosphorsäure, eine Sulfonsäure wie Trifluormethansulfonsäure und insbesondere ein Gemisch aus Trifluormethansulfonsäure/Phosphorsäureanhydrid verwendet. Das Molverhältnis von HMF zu der Säure liegt vorzugsweise zwischen 1 : 0,5 und 1 : 2. Das Molverhältnis von HMF zu dem Nitril liegt vorzugsweise in einem Bereich von 1 : 100 bis 1 : 300.

In der zweiten Stufe wird die N-Acylfuranverbindung (IV) zu der entsprechenden Furanon-Verbindung oxidiert, nämlich zu dem N-Acyl-5-aminomethyl-5-hydroxydihydro-2,5-furan-2-on der allgemeinen Formel V:

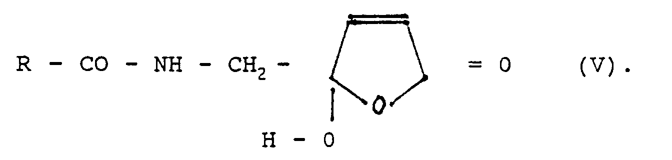

Die Oxidation der entsprechenden N-Acylfuranverbindung (IV) wird durch Photooxidation mittels Sauerstoff in Gegenwart eines Photosensibilisators, insbesondere mit auf einem Harz niedergeschlagenen Bengalrosa durchgeführt. Besonders geeignet ist hierfür ein Sephadex QAE 25 Harz (L. Cottier et al., Bull. Soc. Chim. Fr. 1986, 848-851), bei dem die Bengalrosa Konzentration etwa 10% beträgt. Diese Oxidation wird bei einer Temperatur zwischen 0 und 50° C in einem polaren Lösungsmittel wie Methanol, Ethanol, Aceton, Dichlormethan oder Tetrahydrofuran duchgeführt.

In der dritten Stufe wird das derart oxidierte Furanon der allgemeinen Formel V zu dem entsprechenden N-Acylderivat der ALA gemäß allgemeiner Formel I reduziert.

Diese Reduktion des rohen Furanons erfolgt vorzugsweise mit Zink in Essigsäure bei einer Temperatur zwischen 0 und 60° C, wobei die heterogene Mischung einer Ultraschallbehandlung unterworfen wird.

Das erfindungsgemäße Verfahren erlaubt es, die N-Acylderivate der ALA in drei Stufen mit einer auf HMF bezogenen Gesamtausbeute zwischen 20 und 60% herzustellen.

Durch saure Hydrolyse der N-Acylderivate der allgemeinen Formel I erhält man mit Salzsäure das Hydrochlorid der ALA.

### Beispiel 1

### A. Herstellung der N-Acylfuranverbindung (IV)

Zu einer Lösung von 0,204 g HMF (1,62 mMol) in 13 ml Acetonitril, die bei 25° C gehalten wird, werden 0,354 ml eines äquimolaren Gemisches von Wasser (3,24 mMol) und Trifluormethansulfonsäure (3,24 mMol) zugetropft. Nach 22 Stunden wird die Lösung mit 20 g Eis verdünnt, mit Natriumhydrogencarbonat neutralisiert und fünfmal mit je 20 ml Dichlormethan extrahiert. Nach Trocknen über Natriumsulfat wird das Lösungsmittel abgedampft. Das Rohprodukt wird chromatographisch über Kieselgel gereinigt und ergibt 120 mg (0,72 mMol) N-Acetyl-5-aminomethylfurfural (IV) entsprechend einer Ausbeute von 47 %, sowie 14 mg nicht umgesetztes HMF, so daß die Gesamtumsetzung 93 % beträgt.

### B. Herstellung der Furanon-Verbindung (V)

Eine Lösung aus 10 ml Ethanol und 405 mg (2,4 mMol) des so erhaltenen N-Acetylderivates (IV) und 150 mg Bengalrosa auf einem Sephadex QAE 25 Harz in 10%iger Konzentration wird unter Sauerstoff mit einer Halogenlampe von 150 W bestrahlt. Nach Absorption des theoretischen Volumens von 54 ml Sauerstoff wird nach etwa vier Stunden die Lösung zwölf Stunden ruhen gelassen und danach filtriert. Nach Verdampfen enthält das Rohprodukt 414 mg N-Acetyl-5-aminomethyl-5-hydroxydihydro-2,5-furan-2-on (V) bzw. 1-N-Acetyl-aminomethyl-1-hydroxy-butenolid, was einer Ausbeute von 64 % entspricht.

### C. Herstellung des Acetylderivates der ALA

Das so erhaltene Furanon-Rohprodukt wird zu 10 ml Essigsäure, welche 966 mg Zinkpulver enthält, gegeben. Die Mischung wird einer Ultraschallbehandlung (160 W, 42 kHz) ausgesetzt, nach zwei Stunden wird filtriert und eingeengt. Der Rückstand wird chromatographisch an Kieselgel gereinigt und ergibt 243 mg (1,35 mMol) N-Acetyl-5-aminolävulinsäure, was einer Ausbeute von 55 % entspricht.

Die Gesamtausbeute bezogen auf umgesetztes HMF entspricht 24 %.

### Beispiel 2

Die derart erhaltene N-Acetyl-5-aminolävulinsäure wurde unter Rühren mit Salzsäure etwa sechs Stunden unter Rückfluß behandelt, abgekühlt, filtriert und unter Vakuum eingeengt. Der aus Ethanol umkristallisierte Rückstand ergab das Hydrochlorid der 5-Aminolävulinsäure in Form einer rötlich kritallinen Masse mit einem Schmelzpunkt von 146-147° C und Analysenwerten von C : 35,6; H : 6.02 ; N : 8,35 (theoretisch für C₅H₁₀NO₃Cl⁻/C : 35,83; H : 6,01 ; N : 8,36).

## Patentansprüche

1. Verfahren zur Herstellung von N-Acylderivaten der 5-Aminolävulinsäure (ALA) der allgemeinen Formel I
R - CO - NH - CH₂ - CO - CH₂ - CH₂ - COOH (I),
in der R Methyl, Ethyl, Propyl, Isopropyl, Butyl, Phenyl, Benzyl, Furyl oder Furfuryl bedeuten, sowie des Hydrochlorids der freien Säure durch saure Hydrolyse, **dadurch gekennzeichnet**, daß man 5-Hydroxymethylfurfural (HMF) der Formel II 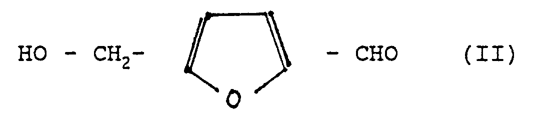 mit einem Nitril der Formel III
R - C ≡ N (III),
in der R die obige Bedeutung hat, mit einem sauren Katalysator zu einem N-Acyl-5-aminomethylfurfural der allgemeinen Formel IV 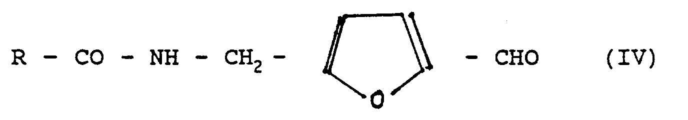 umsetzt, in der R die obige Bedeutung hat, diese mittels Photooxidation in ein N-Acyl-5-aminomethyl-5-hydroxydihydro-2,5-furan-2-on der Formel V umwandelt 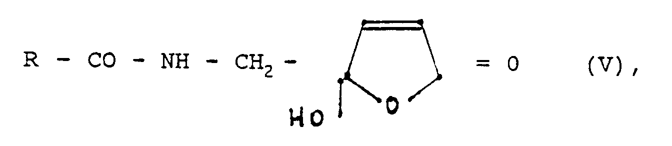 in der R die obige Bedeutung hat, und dieses Furanon durch Reduktion in die N-Acyllävulinsäure der Formel I überführt.

2. Verfahren nach Anspruch 1, **dadurch gekennzeichnet**, daß man das Acylderivat der allgemeinen Formel
R - CO - NH - CH₂ - CO - CH₂ - CH₂ - COOH (I)
durch saure Hydrolyse mit HCl zu dem Hydrochlorid der 5-Aminolävulinsäure umsetzt.

3. Verfahren nach Anspruch 1 oder 2, **dadurch gekennzeichnet**, daß bei der Kondensationsreaktion von HMF mit dem Nitril als saurer Katalysator eine Mineralsäure, eine Sulfonsäure oder eine Mischung aus Methansulfonsäure und Phosphorsäureanhydrid verwendet wird.

4. Verfahren nach Anspruch 3, **dadurch gekennzeichnet**, daß das Molverhältnis von HMF zu der Säure in einem Bereich von 1 : 0,5 bis 1 : 2 liegt.

5. Verfahren nach Anspruch 3 und 4, **dadurch gekennzeichnet**, daß das Molverhältnis von HMF zu Nitril in einem Bereich von 1 : 100 bis 1 : 300 liegt.

6. Verfahren nach Anspruch 3 bis 5, **dadurch gekennzeichnet**, daß die Kondensationsreaktion bei einer Temperatur zwischen 0 und 50° C durchgeführt wird.

7. Verfahren nach Anspruch 1 bis 6, **dadurch gekennzeichnet**, daß die Photooxidation des N-Acyl-5-aminofurfurals durch Sauerstoff in Gegenwart eines Photosensibilisators und bei einer Temperatur zwischen 0 und 50° C durchgeführt wird.

8. Verfahren nach Anspruch 7, **dadurch gekennzeichnet**, daß die Photooxidation in einem polaren Lösungsmittel wie Methanol, Ethanol, Aceton oder Dichlormethan durchgeführt wird.

9. Verfahren nach Anspruch 7 und 8, **dadurch gekennzeichnet**, daß bei der Photooxodation als Photosensibilisator Bengalrosa in Lösung oder auf einem harzartigen Träger niedergeschlagen verwendet wird.

10. Verfahren nach Anspruch 1 bis 9, **dadurch gekennzeichnet**, daß die Reduktion des N-Acyl-5-aminomethyl-5-hydroxydihydro-2,5-furan-2-on's durch Zink in Essigsäure unter Ultraschallbeschallung bei einer Temperatur zwischen 0 und 60° C durchgeführt wird.
